# EUROPEAN PATENT APPLICATION

(11) **EP 2 395 072 A1**
(43) Date of publication of application: **14.12.2011**
(21) Application number: 11168585.5
(22) Date of filing: 02.06.2011
(51) Int. Cl.: C11D 3/20, C11D 3/22, A61K 8/73, A61K 8/86, A61Q 5/00, A61Q 19/00

(54) **Improved cleaning formulations**

(30) Priority: 11.06.2010 US 397444 P
(71) Applicant: Dow Global Technologies LLC, Midland, MI 48674 (US); Dow Brasil Sudeste Industrial Ltda., Sao Paulo SP (BR)
(72) Inventor: Argenton, Andre, Midland, MI Michigan 48640 (US); Jorge, Alvim, Cep. 04713-002 Sao Paolo (BR); Santos, Antonio Lucio, 04717903 Sao Paulo (BR)
(74) Representative: Buckley, Guy Julian

(57) **Abstract**

The present invention provides mixtures of water soluble polymers for making aqueous cleaning formulations having improved sensorial properties, while maintaining suitable levels of viscosity, foaming, emulsion capacity and cleaning efficiency. The aqueous cleaning formulations may be hand dish washing formulations, as well as laundry detergents and hard surface cleaning formulations.

## Description

### Field of the Invention

The present invention relates to mixtures of water soluble polymers for making aqueous cleaning formulations having improved sensorial properties, while maintaining suitable levels of viscosity, foaming, emulsion capacity and cleaning efficiency. The aqueous cleaning formulations may be hand dish washing formulations, as well as laundry detergents and hard surface cleaning formulations.

### Background of the Invention

Hand dish washing is an activity performed regularly by consumers and typically requires several minutes or even hours. Thus, consumers are in physical contact with dishes and dish washing formulations frequently and sometimes for long periods of time, and even twice or more times in a day.

Dish washing formulations typically include active ingredients such as surfactants and degreasers which perform the cleaning functions, but which are also often harsh and abrasive to consumers' skin. The routine and repeated exposure of consumers to dish washing formulations, therefore, may and often does cause irritation and injury to the consumers' skin. As a result, consumers seek dish washing formulations with superior sensorial properties that don't irritate the skin.

At the same time, however, consumers aren't willing to use a product that can't deliver other important properties of dish washing formulations, such as foaming, suitable viscosity, cleaning efficiency and grease removal, which are typically provided by the surfactants and degreasers. Other properties that should also be maintained in dish washing formulations include viscosity and emulsion capacity. It is also, obviously, helpful to reduce the amount of active ingredients, thereby conserving resources and reducing costs, while maintaining or enhancing foaming and cleaning properties.

Various approaches involving changes to the surfactants have been developed to produce hand dish washing formulations with enhanced properties. For example, it is known to simply increase the amount of surfactant in the formulation to achieve improved foaming properties. It is also known to increase the nonionic surfactant content of the formulation to provide an improvement in cleaning efficiency.

Additionally, the industry has developed ways to produce hand dish washing formulations with enhanced sensorial properties while maintaining or enhancing other properties, but these also rely on modifications to the amount, the type, or both, of the surfactant(s) used in the formulations. For example, the typical anionic surfactants may be substituted with alkyl polyglycosides (APGs) which provide better foaming properties and/or less irritation. Also, amphoteric surfactant(s) may be added to the formulation to reduce skin irritation and increase foam stability. Use of extremely mild surfactants such as alkyl polysaccharides, sulphobetaines and synergistic blends of surfactants may beneficially replace usual high levels of an aggressive anionic surfactant. However, greater amounts of the milder surfactants must be used to maintain all desired properties (e.g., foaming, viscosity, etc.).

For example, research reported in Performance optimization in dishwashing liquids containing LABS, alcohol ethersulfates, and alkyl polyglycosides, by Malihi, Farrokh B.; Malihi, Golrokh B. Fargol Research Group, Tehran, Iran; World Surfactants Congress, 5th, Firenze, Italy, May 29-June 2, 2000 (2000), 1317-1324; Publ. Comite Europeen des Agents de Surface et leurs Intermediaires Organiques, Brussels, Belgium, describes investigation of optimal binary and ternary mixtures of anionic and nonionic surfactants for light duty liquid detergents, such as hand dish washing formulations. The surfactants explored include alkylbenzene sulfonate (LABS), alcohol ether sulfates (SLES-2E0), alcohol ethoxylate (AE-7E0 and AE-9E0), and lauryl polyglycoside (APG). Surfactant mixtures having enhanced foaming and cleaning efficiency were reported.

It is known that addition of certain water soluble polymers to hand dish washing formulations containing surfactants will provide improvements to certain properties. For example, high molecular weight polyethylene glycol (e.g., weight-average molecular weight of 600,000 to 4,000,000 Daltons) is useful for improving foaming properties of aqueous surfactant formulations. Also useful for boosting foaming properties in such formulations is hydroxypropyl methylcellulose. Hydroxy ethyl cellulose is known to increase the viscosity of aqueous surfactant formulations. Cationic celluloses are known to be useful in such formulations for improving deposition and foaming characteristics.

European Patent Application EP 0124367 describes hand dish washing formulations comprising dialkyl sulphosuccinates (LABS) as surfactants, along with 0.5 to 5 %, by weight, of a water soluble polymer selected from polysaccharides having hydrophilic substituents (which includes cellulose esters or ethers), xanthan gums, and synthetic polymers having carboxyl substituents. These formulations are asserted to have enhanced foam stability and increased viscosity.

International (PCT) Patent Publication WO2006/076065 discloses personal care compositions containing benefit agents such as perfumes, sun screens, and moisturizers, which are intended to deposit on substrates such as skin and hair. The compositions described in this patent publication contain a low molecular weight surfactant, including anionic, cationic, amphoteric and nonionic surfactants, and a water soluble polymer, such as polysaccharides, gelatins, poly(amino-acids), polylactic acid, and others. The water soluble polymer preferably comprises a polysaccharide, such as, but not limited to, cellulose ether (e.g., METHOCEL). It is asserted that substitution of a portion of the surfactant used in a previously typical formulation with a water soluble polymer such as METHOCEL improves deposition of the benefit agent. This appears to recommend against use of water soluble polymers in systems such as hand dish washing formulations wherein it is sought to improve, or at least leave unchanged, cleaning efficiency, because this would be the *avoidance of deposition* of soil.

Japanese Patent Application JP 02123193A describes mild detergent formulations useful for dishwashing, shampoos and body washes, having reduced irritancy which comprise mild surfactants, such as alkyl glycosides, along with both a polyacrylic polymer (synthetic) and a natural polymer (fully natural). In particular, suitable polymers according to this Japanese patent application include, among others, water soluble xanthan gum, glucomannan, pectin, chondroitin 4-sulfate, chondroitin 8-sulfate, dermatan sulfate, keratan sulfate, heparan sulfate, and partially crosslinked polyacrylic acid and/or salts. Improvements in sensorial properties of the formulations are demonstrated in JP 02123193A when greater amounts of alkyl glycoside surfactants are used in the formulations. Alkyl glycosides are exceptional foaming agents with very low toxicity.

The present invention provides synergistic mixtures of water soluble polymers that, when added in small amounts to aqueous surfactant formulations such as those used for hand dish washing formulations, improve the sensorial properties of the formulation while also maintaining, or even improving, other properties such as viscosity, foaming and cleaning efficiency.

### Summary of the Invention

The present invention provides a mixture of water soluble polymers for use in cleaning formulations having improved sensorial properties, wherein the mixture comprises: (a) 0.5 - 50%, by weight, of high molecular weight polyethylene glycol; (b) 0.5 - 50%, by weight, of hydroxypropyl methylcellulose; (c) 0 - 50%, by weight, of hydroxyethyl cellulose; (d) 0 - 50%, by weight, of hydrophilic cationic cellulose; and (e) 0 - 50%, by weight, of cationic cellulose which is less hydrophilic than polymer (d), based on the total weight of the mixture. Water soluble polymer (a) may, for example, have a weight average molecular weight (MW_{w}) of from 600,000 to 4,000,000 daltons. Furthermore, water soluble polymer (b) may have a MW_{w} of 40,000 to 250,000 daltons, and each of said water soluble polymers (c), (d) and (e) may have a MW_{w} of 20,000 to 200,000 daltons. The mixture of water soluble polymers may, for example, be in a cleaning formulation for hand dish washing.

In one embodiment, the mixture of water soluble polymers comprises: (a) 12% by weight of high molecular weight polyethylene glycol; (b) 48% by weight of hydroxypropyl methylcellulose; and (c) 40% by weight of hydroxyethyl cellulose, based on the total weight of the WSP mixture.

The present invention also provides a method for improving the sensorial properties of a cleaning formulation comprising one or more surfactants which comprises substituting a portion of the total surfactant content of the formulation with the WSP mixture in accordance with the present invention described above, in a ratio of (WSP mixture) : (surfactant content reduction) between 1:6 and 1:60.

The present invention further provides a cleaning formulation comprising: (A) 0.95 - 15 %, by weight, of an active ingredient comprising at least one surfactant; (B) 0.05 - 10 %, by weight, of the mixture of water soluble polymers according to the present invention; and (C) 75 - 99 %, by weight, of water, based on the total weight of the cleaning formulation. For example, the cleaning formulation may comprises (B) 0.05 - 5 %, by weight, of the mixture of water soluble polymers in accordance with the present invention.

In certain embodiments of the cleaning formulation of the present invention, the at least one surfactant is selected from the group consisting of: an alkylbenzene sulfonate, an alcohol ether sulfate, an alcohol alkoxylate, an alkyl polyglycoside, a betaine, a fatty acid amide and an alcohol sulfate.

### Detailed Description of the Invention

The present invention provides mixtures of water soluble polymers ("WSP mixtures") that synergistically improve the sensorial properties of aqueous surfactant formulations while also maintaining, or even improving, other properties of the formulations such as viscosity, foaming and cleaning efficiency.

It should be understood that, although the inventive WSP mixtures and the formulations to which they are applied shall hereinafter be described with focus on formulations suitable for hand dish washing formulations, the present invention is not limited to hand dish washing formulations. In fact, the WSP mixtures may be successfully used in other surfactant and detergent formulations, such as laundry detergents and hard surface cleaning formulations, to improve sensorial, foaming, and other properties.

It has been discovered that, while various water soluble polymers are individually known and used to improve particular properties of aqueous surfactant formulations, certain combinations of such water soluble polymers may be used to replace a portion of the surfactant in such formulations and operate synergistically to improve the sensorial properties of the formulations, while maintaining or even improving the other important properties of the formulations, such as viscosity, foaming and cleaning efficiency.

More particularly, the mixtures of water soluble polymers of the present invention comprise:
(a) 0.5 - 50%, by weight, of high molecular weight polyethylene glycol;
(b) 0.5 - 50%, by weight, of hydroxypropyl methylcellulose;
(c) 0 - 50%, by weight, of hydroxyethyl cellulose;
(d) 0 - 50%, by weight, of hydrophilic cationic cellulose; and
(e) 0 - 50%, by weight, of cationic cellulose which is less hydrophilic than polymer (d),
all weight percentages based on the total weight of the mixture of polymers. Each of the water soluble polymers (a)-(e) will now be more fully defined.

As used herein, high molecular weight polyethylene glycol means a linear homopolymer derived from ethylene oxide and having a weight average molecular weight (MW_{w}) of from 600,000 to 4,000,000 daltons. High molecular weight polyethylene glycol suitable for use in the WSP mixtures of the present invention is, for example, without limitation, commercially available under the trade name POLYOX from The Dow Chemical Company which is headquartered in Midland, Michigan, U.S.A. Use of only high molecular weight polyethylene glycol as the water soluble polymer, in amounts from about 0.1 - 2 % by weight based on the total weight of the cleaning formulation, is known to improve the foaming properties of the formulation.

Hydroxypropyl methycellulose, as used herein, means a methyl ether of cellulose produced from cellulose, methyl chloride and propylene oxide, and having a MW_{w} of from 40,000 to 250,000 daltons. Hydroxypropyl methylcellulose suitable for use in the WSP mixtures of the present invention is, for example, without limitation, commercially available under the trade name METHOCEL from The Dow Chemical Company. Use of hydroxypropyl methycellulose as the only water soluble polymer in amounts from about 0.2 - 2 % by weight based on the total weight of the cleaning formulation is known to improve the foaming properties of the formulation.

As used herein, hydroxyethyl cellulose (HEC) is a water-soluble polymer, also derived from cellulose, and useful for increasing viscosity of aqueous formulations. Hydroxyethyl cellulose suitable for use in the WSP mixtures of the present invention has a MW_{w} of 20,000 to 200,000 daltons and, for example, without limitation, is commercially available under the trade name CELLOSIZE from The Dow Chemical Company. Where HEC is the only water soluble polymer added to the cleaning formulation, in amounts from about 0.3 - 4 % by weight based on the total weight of the cleaning formulation, the viscosity of the cleaning formulation will be increased.

The term cationic cellulose, as used herein, means a straight chain hydroxyethyl cellulose polymer derived from cellulose and having a MW_{w} of 20,000 to 200,000 daltons. These are well-known anti-irritants, but they are also generally known and used for enhancing deposition properties in aqueous formulations. As already described, the surfactant formulations to be improved by the WSP mixtures of the present invention typically benefit from deposition *inhibitors,* not enhancers, since it is efficient removal of soil and grease that is the goal of the aqueous surfactant formulations of concern. Nonetheless, it has been discovered that small amounts of cationic cellulose polymers, in combination with the other water soluble polymers listed above, provides the desired improvement to sensorial properties while not interfering with the cleaning efficiency provided by the surfactants in such formulations as are used for hand dish washing. Use of a cationic cellulose as the sole water soluble polymer, in amounts from about 0.1 - 0.7 % by weight based on the total weight of the cleaning formulation, is known to improve the foaming and deposition properties of the formulation.

The "hydrophilic cationic cellulose" suitable for use in the WSP mixtures of the present invention is basically hydroxyethyl cellulose with cationic substitutions and has a MW_{w} of 20,000 to 200,000 daltons. Such suitable hydrophilic cationic cellulose is, for example, without limitation, commercially available under the trade name UCARE from The Dow Chemical Company.

On the other hand, as used herein, "cationic cellulose which is less hydrophilic than [the hydrophilic cationic cellulose described above]" means hydroxyethyl cellulose having both cationic and hydrophobic substitutions and having a MW_{w} of 20,000 to 200,000 daltons. Such "less hydrophilic" cationic cellulose which is suitable for use in the WSP mixtures of the present invention, is, for example, without limitation, commercially available under the trade name SOFTCAT from The Dow Chemical Company.

Application of any one of the water soluble polymers described above into formulations will deliver single benefits as noted above. However, when used alone, none of these water soluble polymers will provide improved sensorial properties, as well as the same or improved other properties (e.g., viscosity, foaming, cleaning efficiency, etc.), to the same level as the synergystic WSP mixtures of the present invention.

In one version, for example, without limitation, the WSP mixture may comprise (a) 20% by weight of high molecular weight polyethylene glycol and (b) 80% by weight of hydroxypropyl methylcellulose, based on the total weight of the WSP mixture. In another version, the WSP mixtures may comprise (a) 15% by weight of high molecular weight polyethylene glycol, (b) 70% by weight of hydroxypropyl methylcellulose, and (d) 15%, by weight, of hydrophilic cationic cellulose, based on the total weight of the WSP mixture. Still another version of the WSP mixture in accordance with the present invention may comprise (a) 12% by weight of high molecular weight polyethylene glycol, (b) 48% by weight of hydroxypropyl methylcellulose, and (c) 40% by weight of hydroxyethyl cellulose, based on the total weight of the WSP mixture.

The WSP mixtures of the present invention are typically effective when added to aqueous cleaning formulations comprising at least one surfactant, in amounts of from 0.05 to 10 %, by weight, based on the total weight of the formulation. For example, the WSP mixtures may be added to aqueous cleaning formulations in amounts from 0.05 to 5 %, or even 0.1 to 5 %, by weight, based on the total weight of the aqueous cleaning formulation, to provide improvement in the sensorial properties of the formulation, while maintaining or even enhancing other properties such as viscosity, foaming and cleaning efficiency.

In particular, it is noted that a portion of the surfactant content of the formulation may be replaced by the WSP mixture to provide a more pleasant sensorial experience to the consumer (less irritation), improve cleaning efficiency, as well as emulsification power of the cleaning formulation. For example, the WSP mixtures may replace a portion of the active content of the formulation in a ratio of 1:6 (WSP mixture: active content reduction) up to 1:60. Such substitution of active ingredient with the WSP mixture serves to reduce the amount of active ingredient used overall, reducing costs of manufacture, while still providing a formulation product having the desired foaming and cleaning properties, along with improved sensorial properties. As already mentioned, formulations which benefit from addition of the WSP mixtures of the present invention are those used for hand dish washing detergents, however, they may also be successfully be applied in laundry detergent and multipurpose cleaning formulations.

The type of surfactant in the cleaning formulation is not particularly limited. Any of the surfactants known and typically used by persons of ordinary skill in the relevant art to make cleaning formulations for hand dish washing, laundry detergent or hard surface cleaning formulations are suitable for inclusion in the cleaning formulations of the present invention. Such surfactants include, without limitation, sodium lauryl ether sulfates (hereinafter referred to as SLES or alcohol ether sulfates), alcohol propoxylates, linear alkylbenzene sulfonate (hereinafter referred to as LABS or alkylbenzene sulfonate), alkyl phenol ethoxylates and propoxylates, alcohol ethoxylates (AE), alkyl glycosides, alkyl polysaccharides (APG), ethoxylated and propoxylated fatty acid amines, among others. The mixtures of water soluble polymers of the present invention provide most notable improvement to sensorial and other properties when used in hand dish washing formulations which include at least one surfactant selected from the group consisting of: an alkylbenzene sulfonate (LABS), an alcohol ether sulfate (e.g., SLES-2EO, 3EO or 4EO), an alcohol alkoxylate (e.g., 5-12 EO and/or 1-4 PO), an alkyl polyglycoside (APG), a betaine (e.g., fatty acid amide propyl betaines), an amide (fatty acid amides) and an alcohol sulfate. Note that "EO" as used in this context is recognized by persons of ordinary skill in the relevant art to mean "ethylene oxide". Thus, SLES-2EO means sodium lauryl ether sulphate with 2 moles of ethylene oxide. Similarly, persons of ordinary skill recognize "PO" to mean "propylene oxide."

Typically, one or more surfactants are included in the cleaning formulation in amounts up to 20 %, or even 30 %, by weight, based on the total weight of the formulation. Such cleaning formulations may further comprise degreasers and other components, depending on their intended uses. The balance of the formulation, i.e., between 75 and 99% by weight, is typically water.

When compiling the formulation, the WSP mixture may be added either before or after surfactant addition. In one method, the surfactants are added (diluted) to water up to the desired concentration, for example, up to 19% by weight, and then the WSP mixture is added to the formulation under stirring until it is fully solubilized. In another approach, the WSP mixture may be added (diluted) to water up to the desired concentration, for example, up to 1 % by weight, under stirring until fully solubilized, and then the surfactant(s) may be added to the formulation up to the desired concentration thereof.

The present invention further provides a method for improving the sensorial properties of a cleaning formulation comprising one or more surfactants which comprises substituting a portion of the total surfactant content of the formulation with a WSP mixture as described above in a ratio of (WSP mixture) : (surfactant content reduction) between 1:6 and 1:60. For example, where a cleaning formulation usually comprises 25% by weight of surfactants, it may be reformulated and produced comprising 19% by weight surfactants (i.e., 6% less surfactants) and 1% by weight of a WSP mixture in accordance with the present invention as described hereinabove, to produce a cleaning solution containing less surfactants, but having better sensorial properties, as well as undiminished, or even improved, foaming, cleaning efficiency and viscosity properties. As another example, the same cleaning formulation may beneficially be reformulated and produced comprising only 13% by weight surfactants (i.e., 12% less surfactants) and 2% by weight of a WSP mixture.

The method for measuring the weight-average molecular weight (MW_{w}) of water soluble polymers, as this characteristic has been discussed hereinabove and will be discussed in the examples provided hereinafter, is as follows.

All chemicals and pullulan standards, as listed below, were purchased from vendors and were used as received.
1. water (DI water) was filtered through a 0.0002 mm nylon cartridge prior to use;
2. sodium azide, 99.99+% purchased from Sigma-Aldrich;
3. pullulan standard;
4. toluene, HPLC grade from Fisher Scientific;
5. bovine serum albumin (BSA), ~98% monomer from Sigma-Aldrich; and
6. the water soluble polymers to be tested: POLYOX WSR 301, POLYOX WSR N60K, CELLOSIZE QP 100 MH, CELLOSIZE QP 30MH, METHOCEL 40-100, METHOCEL 40-101, SOFTCAT SX 1300X, UCARE Polymer JR30M.

### Mobile phase

The eluent was prepared by dissolving NaN3 in DI water to a concentration of 0.05 wt%, and was filtered through the cartridge. After dissolution it is re-circulated up to 5 hours in the cartridge in order to certify the purity of the solution. It should reach the refractive index of 1.3365 measured by a Wyatt Optilab Rex Dri detector.

### Sample preparation

Samples solutions were prepared in pre-chilled mobile phase to minimize interference. Allowed the solution to shake on a flat-bed shaker for 3 hours to dissolve the sample, placed in refrigerator overnight at 4°C for complete hydration and dissolutions. On the second day, solutions were shaken again for 2 hours. Solutions were filtered through a 0.45 um (micron) nylon syringe filter prior to injection.

The target sample concentrations used are in listed in the table below. The goal was to keep the solution concentration sufficiently below the polymer chain entanglement concentration (C*) to ensure that polymer chain entanglements were minimized. C* is defined as 1/[n] and [n] is calculated according to the approximate weight average molecular weight for polymers - estimated according to the 2% Brookfield solution viscosity data using K= 2.216x10^-4 dl/g and alpha=0.849)

**Table of target concentrations**

| Mw (g/mol) | Target Concentration (mg/ml) |
|---|---|
| 1.00E5 | 0.5 - 1.00 |
| 3.00E5 | 0.3 |
| 5.00E5 | 0.2 |
| 7.00E5 | 0.1-.2 |
| 1.00E6 | 0.10 |

### SEC equipment

Pump: A Waters 2690 pump was set at the optimum flow rate according to the SEC fractionation study and equipped with a filter that consists of two layers of 0.2 microns nylon membrane installed prior to the injection valve.

Injection: The Waters 2690 pump was programmed to inject 100 microliters of solution.

Columns: Two TSK-Gel GMPW columns (7.5mm ID x 30 cm, 17 microns particles, 100 to 1000 angstroms pores nominal) were each operated at 28°C.

Detectors: A Wyatt Dawn DSP MALLS detector connected with a Wyatt Optilab rEX Dri detector was used. The Wyatt DAWN DSP was equipped with a red laser operating at a wavelength of 632.8 nm and operated at room temperature. The Wyatt Optilab rEX DRI detector was operated at 28°C.

Calibration: The Wyatt DAWN MALLS was calibrated with HPLC grade toluene filtered through a 0.05 microns syringe filter. The individual light scattering detectors were normalized to the 90° light scattering detector using mono-dispersed BSA monomer as the isotropic peak with DRI peak of BSA.

### Software and data process

The signals from the MALLS and DRI detectors were analyzed using ASTRA V software. Mw and Rg were determined at each SEC elution volume increment. To obtain these metrics, Debye Plots covering the angular range from 25.8° to 132.2° were least-squares fit to a first order polynomial according to the Zimm formalism. A dn/dc 0.140 mL/g for polymers was applied. Reported molecular weight averages and radii of gyration were based on the first order exponential fitting of slice Mw ASTRA V software. Mass recovery was obtained by comparing the detected mass from integrating of the DRI signal to the injected mass.

Molecular weight equation: Mw = [KC/R0]-1

### EXAMPLES

### WSP Mixture Preparation

Various polymers were measured and combined, in dry powder form to create the blends (mixtures) in the following Table 1. After being measured, each group of powder polymers was added to a V shape powder mixer and allowed to mix for 15 minutes in order to ensure it was thoroughly blended. The quality of the blends may be evaluated by analyzing the viscosity of each blend when solubilized at 2% in water.

**Table 1 - Polymer Blend Compositions**

| | Cellosize QP100MH | Polyox WSR N60K | Methocel 40-101 | Ucare Polymer JR30M |
|---|---|---|---|---|
| Blend 1 | 0 | 20 | 80 | 0 |
| Blend 2 | 0 | 15 | 70 | 15 |
| Blend 4 | 80 | 20 | 0 | 0 |
| Blend 5 | 40 | 12 | 48 | 0 |

### Addition of Each Mixture/Blend to A Hand Dish Washing Formulation

Each individual type of water soluble polymer, as well as each of Blends 1, 4 and 5, was added to each of four cleaning formulations comprising 15% of linear alkyl benzene sulfonate sodium (in active basis), also known as LABS, and 5% of lauryl ether (2 ethylene oxide moles) sulphate sodium (in active basis), also known as SLES, as the surfactant, also added 2% of sodium chloride, in dry form, to produce the formulations listed in the following Table 2. In each case, the surfactant was added (diluted) to water up to the desired concentration, as noted in the table, and then the polymer or blend was added to the formulation under stirring until it fully solubilized. All formulations were stable after 4 weeks at 54°C.

The viscosities for each formulation are also provided in Table 2. In Formulation A, the surfactant content was reduced from the initial 20% by weight in the Initial Formulation (column 1) to 15% by weight, and it can be seen in Table 2 that the reduction in surfactant concentration resulted in reduced viscosity compared with a full 20% by weight load of surfactant. It is also apparent from the data reported in Table 2 that the addition of individual polymers to formulations having lower surfactant content (columns 3 and 4, rows 1 to 4) resulted in increased viscosities, but none of the independent polymers is able to restore the same degree of viscosity as certain of the polymer blends are able to, see, e.g., formulations containing polymer blend 4 or 5.

**Table 2 - Viscosities of Cleaning Formulations**

| Formulation WSP/Blend | Viscosity (RVT sp2) | | | |
|---|---|---|---|---|
| | **Initial Formulation** (20% surfactant) | **Formulation A** (15% surfactant -no blend) | **Formulation B** (13.5% surfactant + 0.5% blend) | **Formulation C** (15% surfactant + 0.5% blend) |
| | Column 1 | Column 2 | Column 3 | Column 4 |
| METHOCEL 40-101 | 830 | 115 | 535 | 615 |
| POLYOX WSR N60K | 830 | 120 | 230 | 415 |
| CELLOSIZE QP100MH | 830 | 130 | 630 | 650 |
| UCARE Polymer JR30M | 830 | 150 | 455 | 515 |
| Blend 1 | 830 | 130 | 300 | 760 |
| Blend 4 | 830 | 145 | 770 | 810 |
| Blend 5 | 830 | 125 | 650 | 815 |

### Evaluation of Hand Dish Washing Formulations

After obtained a stable formulation it is required to assess its performance against a benchmark, which in this case was the Initial Formulation, which contained 20% by weight surfactant(s).

The first evaluation is the emulsifying power (i.e., cleaning efficiency), simulate the consumer hand dishing washing process, and it is evaluated by the following procedure:
1. Soil standardized 20 cm diameter dishes with 1 g of grease soil. Grease soil is composed of 1 part of animal fat and 1 part of vegetable oil.. Typically 300 to 400 plates are soiled at the same time
2. Allow those soiled plates to stand for one day at 22°C.
3. Dilute 3g of formulation to be tested in 200g of water (this is the testing solution).
4. The testing solution is manually agitated in an open container with a typical urethane-based sponge to generate foam.
5. The test consists of washing as many plates as possible with aforesaid foam, until it is impossible to obtain more foam
6. The number of plates washed is reported.

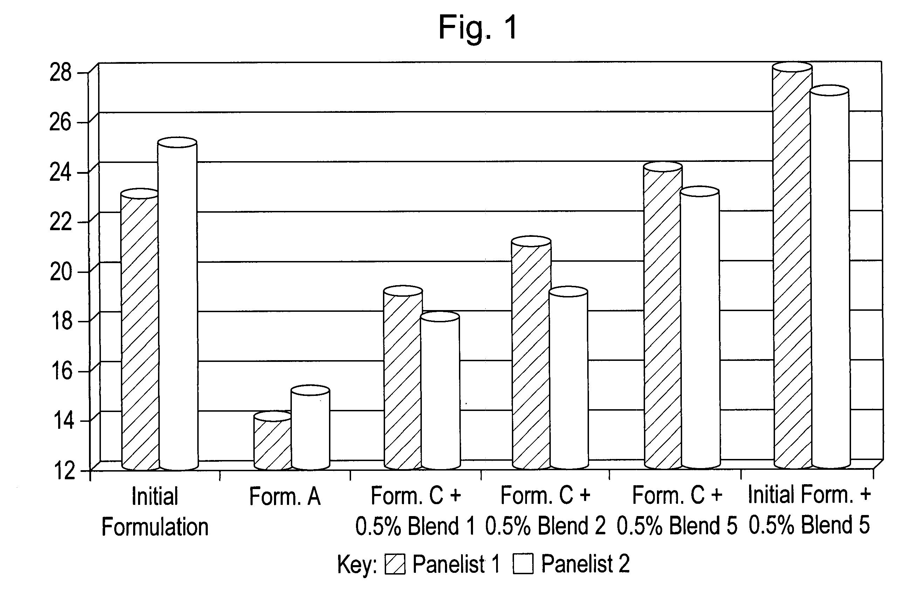

This process is designed to provide information concerning the cleaning efficiency of each formulation. In order to obtain a good statistical result, the foregoing process (1-6) was performed independently by two panelists, and each panelist repeated the process 3 times. The results of this evaluation are provide in the chart in Figure 1.

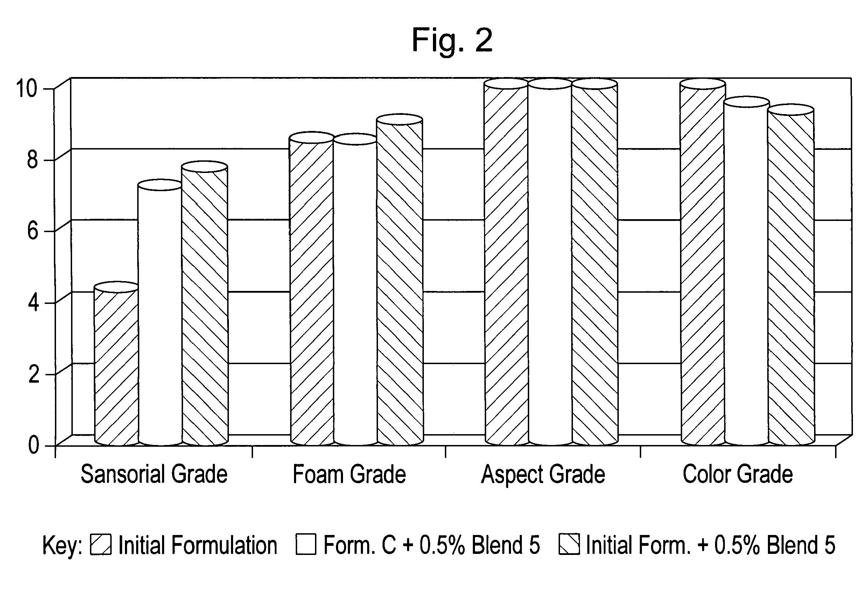

The other important evaluation is the sensorial properties of each formulation, as perceived by the user/panelist. In this subjective test, the panelist was asked to assign a grade of from 1 to 10, to each formulation, for each of the following parameters:
Sensorial score: after washing 3 plates, each panelist was asked what was the
   overall sensorial quality of the process, higher grades mean better or more pleasant sensorial.
Foam score: after washing 3 plates, each panelist was asked what was the
   overall foaming quality during the process, higher grades mean better or more foam.
Visual Appearance score: evaluating the formulation's visual appearance in a
   standard detergent bottle, the closer to standard higher grades.
Color score: compare the color of each formulation against a standard to provide
   indication of relative turbidity and intensity.
10 panelists performed this evaluation and the results are shown in the chart in Figure 2. The values reported in Figure 2 are the average values of the 10 panelists for each feature for each formulation.

As seen in Figure 2, both formulations containing polymer Blend 5 were reported to have better, more appealing sensorial qualities than the formulation without any polymer. Each formulation received similarly high grades for each of the other attributes, i.e., Foam, Visual Appearance and Color.

## Claims

1. A mixture of water soluble polymers for use in cleaning formulations having improved sensorial properties, said mixture comprising:
(a) 0.5 - 50%, by weight, of high molecular weight polyethylene glycol;
(b) 0.5 - 50%, by weight, of hydroxypropyl methylcellulose;
(c) 0 - 50%, by weight, of hydroxyethyl cellulose;
(d) 0 - 50%, by weight, of hydrophilic cationic cellulose; and
(e) 0 - 50%, by weight, of cationic cellulose which is less hydrophilic than polymer (d),
based on the total weight of the mixture.

2. The mixture of water soluble polymers according to Claim 1, wherein said water soluble polymer (a) has a weight average molecular weight (MW_{w}) of from 600,000 to 4,000,000 daltons.

3. The mixture of water soluble polymers according to Claim 1, wherein said water soluble polymer (b) has a MW_{w} of 40,000 to 250,000 daltons, and each of said water soluble polymers (c), (d) and (e) has a MW_{w} of 20,000 to 200,000 daltons.

4. The mixture of water soluble polymers according to Claim 1, wherein said cleaning formulation is a hand dish washing formulation.

5. The mixture of water soluble polymers according to Claim 1, comprising:
(a) 12% by weight of high molecular weight polyethylene glycol;
(b) 48% by weight of hydroxypropyl methylcellulose; and
(c) 40% by weight of hydroxyethyl cellulose,
based on the total weight of the WSP mixture.

6. A method for improving the sensorial properties of a cleaning formulation comprising one or more surfactants which comprises substituting a portion of the total surfactant content of the formulation with the WSP mixture of Claim 1, in a ratio of (WSP mixture) : (surfactant content reduction) between 1:6 and 1:60.

7. A cleaning formulation comprising:
(A) 0.95 - 15 %, by weight, of an active ingredient comprising at least one - surfactant;
(B) 0.05 - 10 %, by weight, of the mixture of water soluble polymers of Claim 1; and
(C) 75 - 99 %, by weight, of water,
based on the total weight of the cleaning formulation.

8. The cleaning formulation according to Claim 7, comprising:
(B) 0.05 - 5 %, by weight, of the mixture of water soluble polymers of Claim 1.

9. The cleaning formulation according to Claim 7, wherein said cleaning formulation is a hand dish washing formulation.

10. The cleaning formulation according to Claim 9, wherein said at least one surfactant is selected from the group consisting of: an alkylbenzene sulfonate, an alcohol ether sulfate, an alcohol alkoxylate, an alkyl polyglycoside, a betaine, a fatty acid amide and an alcohol sulfate.
